(19) Europäisches Patentamt
European Patent Office
Office européen des brevets

(11) EP 3 878 454 A1

(12) EUROPEAN PATENT APPLICATION
published in accordance with Art. 153(4) EPC

(43) Date of publication:
**15.09.2021 Bulletin 2021/37**

(21) Application number: **19882497.1**

(22) Date of filing: **19.09.2019**

(51) Int Cl.:
***A61K 31/566*** *(2006.01)* ***A61P 35/00*** *(2006.01)*
***C07J 63/00*** *(2006.01)*

(86) International application number:
**PCT/RU2019/000650**

(87) International publication number:
**WO 2020/096486 (14.05.2020 Gazette 2020/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.11.2018 RU 2018139336**

(71) Applicant: **Iliyasova, Natalia Eduardovna**
**Sankt-Peterburg, 197022 (RU)**

(72) Inventors:
- **ILIYASOV, Shamil Sionovich**
  **St.Petersburg, 192022 (RU)**
- **SHAVVA, Alexandr Grigorievich**
  **St.Petersburg, 192029 (RU)**
- **MOROZKINA, Svetlana Nikolaevna**
  **St.Petersburg, 198504 (RU)**

(74) Representative: **KATZAROV S.A.**
**Geneva Business Center**
**12 Avenue des Morgines**
**1213 Petit-Lancy (CH)**

# (54) USE OF 3-O-SULFAMATE-16,16-DIMETHYL-D-HOMOEQUILENIN TO TREAT ONCOLOGICAL DISEASES

(57) The invention relates to the field of medicine and to the chemical and pharmacological industry, and concerns agents for the treatment of cancer. 3-O-Sulfamate 16,16-dimethyl-D-homoequilenin is proposed as an anti-cancer agent in monotherapy and adjuvant therapy of oncological diseases such as hepatocellular carcinoma, gastric carcinoma, lung cancer, chronic myelogenous leukemia, and breast cancer including triple negative breast cancer. The new compound has not any uterotropic activity in animals and does not influence on endometrium.

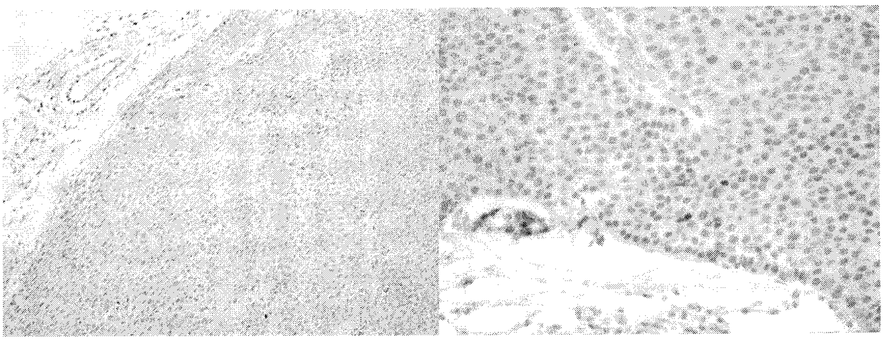

Fig. 1

EP 3 878 454 A1

## Description

### TECHNICAL FIELD

The invention relates to the field of medicine and to the chemical and pharmaceutical industry and concerns medicaments for the treatment of cancers including breast cancer.

### BACKGROUND OF INVENTION

Breast cancer is the leading oncological disease in women [Parkin D.M., Bray F., Ferlay J., Pisani P., CA Cancer J. Clin., 2005, vol. 55, p. 74-108.]. According to the WHO data, there are 2.09 million patients worldwide with annual breast cancer-related mortality of 627 thousand women every year [http://www.who.int/news-room/fact-sheets/detail/cancer].

A significant portion of tumors with this localization progresses under the action of estrogens [Yue W., Yager J.D., Wang J.-P., Jupe E.R., Santen R.J., Steroids, 2013, vol. 78, p. 161-170.]. Immunohistochemical analysis reveals breast cancers that express receptors for estrogen (ERs), progesterone (PRs) and HER2NEU (which renders the cancer "herceptin sensitivity"). A significant portion of tumors have ERs, PRs and/or HER2NEU 3+. If a certain tumor has no ERs and PRs and is not sensitive to Herceptin (ER0, PR0, HER2NEU 0-1), it is considered as a triple negative form (ER-/PR-/HER-2-). This is one of the most lethal forms of breast cancer as it has no targets for the inhibition of its growth. Currently, there are no medicaments for the treatment of this form of cancer in the world.

Estrogens circulate in the blood and accumulate in tumors in the form of sulfates, which are unable to bind to estrogen receptors however after being converted into free hormones they activate tumor growth. Therefore, a promising one is a strategic line of the treatment comprising using inhibitors blocking the formation of this group of free hormones in a tumor.

Tamoxifen is a medicament from the group of selective estrogen receptor modulators, which blocks estrogen effects on hormone-dependent tissues including breast tissue. Tamoxifen is a standard of hormone therapy for premenopausal and postmenopausal women. The most typical side effects which develop during the use of tamoxifen include: an increased risk of venous thrombosis, aggravation of the course of cardiovascular diseases (including angina pectoris attacks), development of endometrial neoplasms (polyps and endometrial cancer), as well as uterine fibroma. Tamoxifen is also hepatotoxic. According to the biopharmaceutical classification system (BCS), developed by Gordon Amidon et al. in 1995, tamoxifen is assigned to the second class, i.e. a medicament with low solubility and high permeability.

Aromatase inhibitors have been shown to be more effective than Tamoxifen. Currently, aromatase inhibitors such as letrozole and anastrazole have been put into use in this field. They are assigned to the first class according to the BCS system, which stands for medicaments with high solubility and high permeability. The side effects of aromatase inhibitors include osteoporosis, hot flashes, and headaches.

In addition to the aforesaid, steroid sulfatase is a subject of great attention due to the local interstitial formation of estrogens from the abundant pool of circulating estrone sulfate. Steroid sulfatase catalyzes the hydrolysis of estrone sulfate to estrone and DHEA sulfate to DHEA (Dibbelt I., Biol. Chem., Hoppe-Seyler, 1991, vol. 372, p. 173-185.; Stein C., J. Biol. Chem., 1989, vol. 264, p. 13865-13872.).

The most known steroid sulfatase inhibitor is EMATE - estrone sulfamate (Ahmed S., Curr. Med. Chem., 2002, vol. 9, no. 2, p. 263-273.). However it has a significant disadvantage. Estrone sulfatase inhibitors containing a sulfamate group cause an irreversible enzyme deactivation with the release of a free ligand [Howarth N.M., Purohit A., Reed M.J. J. Med. Chem., 1994, vol. 37, p. 219-221.]. In particular, estrone sulfamate inhibits estrone sulfatase but the release of free hormone leads to the emergence of strong uterotropic activity [Shields-Botella J. et al., J. SteroidBiochem. Mol. Biol., 2003, vol. 84, p. 327-335.].

Therefore when searching for new anti-cancer agents it is necessary to take into account that a carrier of the sulfamate group of the estrone sulfatase inhibitor should not possess hormonal (uterotropic) activity.

D-equilenin devoid of cancerogenic properties is known to be used as a medicament for the prevention of breast carcinoma. Preparations with hypocholesterolemic action having no uterotropic and hypertriglyceridemic activities have been prepared from equilenin derivatives [Urusova E.A., Gluzdikov I.A., Selivanov S.I., Starova G.L., Nikolaev S.V., Shavva A.G. Synthesis and study of equilenin derivatives and its modified analogs. Russ. J. Org. Chem. 2004, Vol. 40, Issue. 4, p. 506-512.].

Equilenin compounds are components of the medicaments (Premarin®) used in hormone replacement therapy. These properties are desirable because the use of inhibitors of steroid estrogens metabolism implies their long-term application.

**DESCRIPTION**

A technical problem of the present invention is to provide compounds and develop an efficient method of synthesis thereof, which compounds could be used as anti-cancer agents in monotherapy and adjuvant therapy of oncological diseases such as hepatocellular carcinoma, gastric carcinoma, lung cancer, chronic myelogenous leukemia, and breast cancer including its most lethal form such as triple negative breast cancer (ER-/PR-/HER-2-).

The problem is solved by a use of the compound of formula (3) named 3-O-sulfamate-16,16-dimethyl-D-homoequilenin, which inhibits estrone sulfatase.

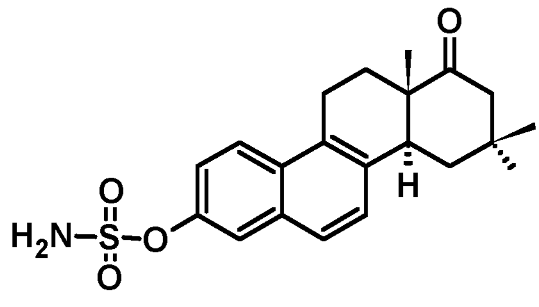

3

The scheme for the synthesis of the target compound of formula (3) is shown below. In contrast to the prior art, a distinguishing advantage of the proposed scheme is that it allows for scaling up the target compound (medicament) production to industrial scale.

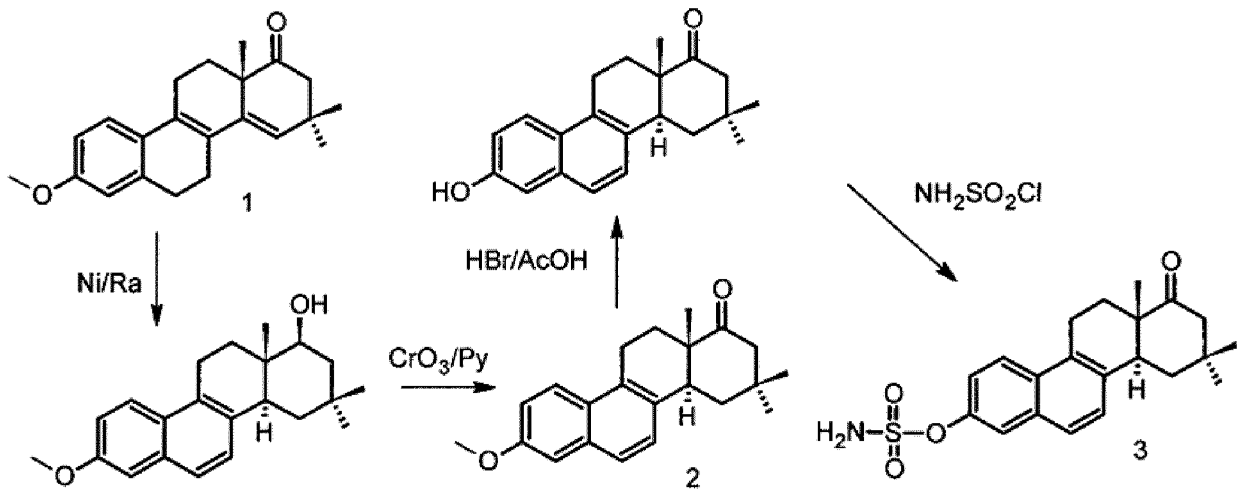


The reaction of the known isothiuronium salt [Al Safar, Zakharychev A.V., Ananchenko S.N., Torgov I.V. Synthesis of some 16,16-dimethyl-D-homosteroids. Reports of Academy of Sciences of the USSR. Ser. Chem., 1968, No. 10, p. 2326-2332.] with 2,4,4-trimethylcyclopentane-1,3-dione leads to the formation of the secosteroid, the cyclodehydration of which yields estrapentaene of formula (1). Having treated estrapentaene by Ni/Ra under the conditions proposed previously for the synthesis of structurally similar D-homoanalogue [Gluzdikov I.A., Egorov M.S., Selivanov S.I., Starova G.L., Shavva A.G. New analogues of D-homoequilenene with substituents in the D ring. Rus. J. Org. Chem., 2006, vol. 42, no. 11, p. 1675-1682.], methyl ester of 16.16-dimethyl-D-homoequilenin of formula (2) was isolated [Gluzdikov I.A. PhD Thesis in Chemistry, 2007, St. Petersburg.]. The sulfamate of formula (3) was obtained according to the well-known method [Morozkina S.N., Gluzdikov I.A., Drozdov A.S., Selivanov S.I., Kovalev R.A., Filatov M.V., Shavva A.G. Russ. J. Org. Chem., 2015, vol. 51, No. 3, p. 425-430.].

The results of the steroid binding simulation have revealed that the structure of this steroid is poorly compatible with the geometry of ligand-binding receptor pocket. The distance between the oxygen in the steroid ketone group and NH of His524 is 4.9 Å, which precludes the formation of a hydrogen bond. The distance between the hydroxyl group at C3 and oxygen atom in the carboxyl group Glu353 is calculated to be about 3.9 Å, which is greater by far than that in the complex with the natural hormone estradiol (2.34 Å).

The incompatibility between the steroid's geometry and the receptor region responsible for ligand binding will

cause low affinity of the subject compound to the receptor resulting in the absence of uterotropic activity of this steroid.

The compound completely inhibits proliferation of tumor MCF-7 cells.

The medicament belongs to the $4^{th}$ hazard class ($LD_{50}$>300-2000 mg/kg). The obtained data allows to acknowledge the doses used for the study of antitumor activity as safe.

The study of binding to the estrogen receptors has shown that sulfamate lacks hormone activity at concentrations of -0.001 and 0.01, 0.1 and 1.0 μmol/ml.

In animal models with xenograft human tumor of triple negative breast cancer, the claimed compound (medicament) is more effective than tamoxifen and aromatase inhibitor letrozole used in clinical practice.

Therefore, the compound can be used in the field of breast cancer treatment including the triple negative form thereof.

Preclinical studies of specific activity in breast cancer models were carried out in parallel comparison with the antitumor medicament Tamoxifen (the reference drug). A comparative study of the medicaments *in vivo* was performed in female Balb/c nude mice with subcutaneously (s.c.) transplanted tumor cells. The experiment lasted for 29 days after the medicament administration for the first time (the administration of the medicaments was started on Day 5 after the tumor strain transplantation).

The medicament and the reference drug were administered intragastrically multiple times at doses of 30 mg/kg and 10 mg/kg daily for 21 days.

The placebo group was given 1% starch solution intragastrically in equivalent volumes and in the same regime. Tumor nodes were measured 2 times per week. The antitumor activity was estimated by the standard tumor growth inhibition (TGI) index. Once the treatment was completed, the last tumor measurement was carried out, and animals were weighed and euthanized.

All necessary manipulations with animals (tests, weighing, measuring, and necropsy of animals) were performed 9 to 12 a.m.

The animals were weighed on the first day of the experiment, and then 2 times per week throughout the experiment according to the research protocol.

A tumor node was measured after tumor transplantation 2 times per week throughout the experiment. The tumor node volume was determined by the formula:

$$V=\pi/6*L*W*H,$$

where L, W, H are the linear size dimensions of the tumor.

*Determination of antitumor activity*

Tumor growth inhibition index (TGI) was used as a criterion for the study of the medicament efficacy and calculated by the formula as follows:

$$TGI(\%)=|V_{ctrl} - V_{exp}|/V_{ctrl}*100$$

where $V_{ctrl}$ and $V_{exp}$ are the average tumor volume ($mm^3$) in the control and in the experimental groups, respectively.

After the course of the treatment, the animals were euthanized by the cervical dislocation of cervical vertebrae.

The statistical data processing was conducted using SPSS 21 package (license No. 20130626-3). Descriptive statistics in the study includes the following: arithmetic average, standard deviation (SD), standard mean error (SE), median, quartiles, interquartile range. To compare quantitative characteristics in the groups, the Mann-Whitney-Wilcoxon test was used without corrections for multiple comparisons. The results were considered statistically significant at $p<0.05$.

Breast tumors (BT) in female FBV/N mice transgenic for HER-2/neu are adenocarcinomas characterized by a low amount of estrogen receptors (ER) (Fig. 1) and lack of progesterone receptors (PR). Two adenocarcinoma subtypes can be differentiated, in particular ER+, PR-, and ER-, PR- [2]. The immunohistochemical analysis of ERα in breast tumors has revealed single positively stained nuclei only in 10 out of 45 samples (Fig. 1).

The model is sensitive to the CAF therapy (cyclophosphamide, adriamycin, 5-fluorouracil) with the observable tumor growth inhibition resulting in the stabilization of the average tumor volumes (Fig. 2). The tumor growth inhibition was 63% on Day 14 and 46% on Day 21 of the experiment.

As animals may develop multiple tumors, it is considered reasonable to analyze an average total tumor volume in mice (Fig. 3) and its relative change versus tumor volume at the beginning of the experiment (Fig. 4). The graph of the relative increase in tumor volume shows a linear change of this parameter in the control group suggesting that this parameter is appropriate to use for assessing a biological response of tumor to the treatment. For instance the treatment

with the CAF therapy does not cause any changes in this parameter for almost 3 weeks indicating disease stabilization. The frequency of individual tumor stabilizations is about 60% by Day 21.

The study was carried out in female FVB mice transgenic for HER-2/neu by conventional category; the strain was obtained from the mouse bank Charles River Laboratories (Italy). The age of animals at the beginning of the study was 21-41 weeks and weight was 25-30 g.

Tamoxifen Hexal (Hexal AG, Germany, 83607 Holzkirchen, lot HA1575 on 01/17) representing a dosage form of the medicament formulated as white or slightly yellowish coated round biconvex tablets, with uniform smooth surface, comprising 20 mg of the active compound was used for the experiment.

The housing conditions were chosen according to the standards specified in The Guide for Care and Use of Laboratory Animals (ILAR publication, 1996, National Academy Press, 1996).

The animals were housed in a separate room in groups (no more than 5 animals in a group) in individual T2 type cages suitable for laboratory rodents. The dimensions of the cages were 268 x 215 x 141 $mm^3$ (base area 370 $cm^2$) each equipped with a polycarbonate tub, stainless steel lid with a feed container, and divider for drinking bottle. The cage bedding was made of dust-free wood shavings.

The animals had unrestricted access to the combined complete pelleted feed for laboratory rodents.

Drinking water was given *ad libitum* in standard 190 ml drinking bottles manufactured by TECNIPLAST, made of high-temperature polysulfone with a silicone ring and a metal lid made of AISI 316 stainless steel.

The room temperature was maintained at a level of 20-26°C with the relative humidity of 50-70%. The photoperiod was set at 12:12 hrs night/day under artificial lighting with fluorescent lamps.

An identification card was placed on the housing cage with a cage/card number, experimental group number, individual numbers of the animals, their number and sex, study number, name of the responsible researcher. The auricle of each animal was clipped with a metal marker for small laboratory rodents made of Kent-Scientific nickel alloy, USA (markers have three-digit numbers stamped by the manufacturer).

The mice were euthanized with carbon dioxide at the end of the experiment. All animal corpses were autopsied followed by microscopic description.

$LD_{50}$ of the medicament was determined in FVB mice transgenic for HER-2/neu using the OECD 423 test (OECD guideline for testing of chemicals. Acute Oral Toxicity - Acute Toxic Class Method).

12 female mice were used in the experiment.

At the first step, 3 mice were given the medicament at a dose of 300 mg/kg. The study medicament was administered intragastrically (i.g.) using a metal atraumatic gavage (the procedure corresponds to the oral administration in clinical settings). For i.g. administration the active compound of the medicament was mixed with olive oil to prepare the suspension of the required concentration for the administration 0.1 ml of the suspension per 10 g of an animal body weight. The dosage form for administration was prepared *ex tempore.*

As no animals died after administration of the medicament at a dose of 300 mg/kg, the same dose was administered to 3 additional animals. No deaths were detected either. Therefore, the medicament was further administered at the dose of 2000 mg/kg in 2 steps, 3 animals per each step.

The clinical observation and registration of body weight were made according to the scheme as shown in Table 1. The animals were examined 2 times a day, clinically examined individually after the dose administration during the first 60 minutes, with special attention during the first 4 hours, periodically during the first 24 hours, on Day 2 and weekly for up to 14 days. Each animal was thoroughly examined in the housing cage in the observer's hands. The overall conditions of the animals were recorded, such as peculiarities in their behavior, intensity and nature of motor activity, fur and skin appearance.

The body weights of the animals were recorded using a verified high-speed electronic laboratory balance Ohaus Scout Pro (USA) with a maximum load of 2000 g and a measurement step of 0.1 g.

On Day 15, the animals were euthanized and undergone autopsy analysis with the registration of macroscopic changes. The data was collected in a special individual autopsy form.

All of the experimental animals were subject to pathomorphological examination at the end of the study. The euthanized animals were carefully examined for the external pathological changes. The chest and abdominal cavities were examined along with the macroscopic analysis of internal organs. The microscopic analysis of organs and tissues of laboratory animals was not carried out, because no deaths of animals were recorded over the whole observation period. In addition, the macroscopic analysis of the internal organs did not reveal any pathological changes.

Table 1.

| Scheme of observation and weighing of animals when assessing acute toxicity | | | | | | | | | | | | | | | |
|---|---|---|---|---|---|---|---|---|---|---|---|---|---|---|---|
| Parameters | Day of experiment (weighing on Day 1 is conducted before the medicament administration) | | | | | | | | | | | | | | |
| | 1 | 2 | 3 | 4 | 5 | 6 | 7 | 8 | 9 | 10 | 11 | 12 | 13 | 14 | 15 |
| Weighing | | | | | | | | | | | | | | | |
| Administration of medicaments | | | | | | | | | | | | | | | |
| Clinical examination | | | | | | | | | | | | | | | |
| Euthanasia of animals | | | | | | | | | | | | | | | |

The study medicament was administered i.g. using a metal atraumatic gavage (with the procedure corresponds to the oral route of administration in clinical settings). The medicament was administered in a single daily dose (20 mg/kg of body weight) and 5-fold daily dose of 100 mg/kg of body weight. *Ex tempore* solutions of the medicament for administration were prepared in olive oil (refined olive oil supplemented with unrefined extra virgin olive oil available under Global Village "Clasico" trademark, lot L:183351116, expiry date 26.04.2020, BAIEO, Spain). The administered volume was 0.1 ml per 10 g of a mouse body weight (0.2 ml of a ready-to-use formulation for a mouse of 20 g). The medicament administration was started 24 hours after the randomization. The duration of the administration course was 27-28 days.

The reference drug, Tamoxifen, was also administered using a metal atraumatic gavage (the procedure corresponds to the oral administration in clinical settings), at a daily dose of 4.0 mg/kg calculated on the basis of the clinical doses [5]. This dose corresponds to a human daily dose of 20 mg/kg. A tamoxifen tablet was crushed in a pounder, and the resulting powder was used to prepare a suspension in 40 ml of the olive oil, the administered volume was 0.08 ml per 10 g of a mouse body weight, the duration of administration was the same as that of the experimental medicament and last for 27-28 days.

The control groups were given the olive oil (placebo).

The evaluation criteria included the clinical observation data, animals body weights, time of death (if applicable), tumor growth over time, pathomorphological examination data (verification of a neoplasm during autopsy, assessment of toxic effects by macroscopic presentation of changes in the internal organs).

The animals body weights were recorded before the first administration of the medicaments and then twice per week using a verified high-speed electronic laboratory balance Ohaus Scout Pro (USA) with a maximum load of 2000 g and a measurement step of 0.1 g.

Macroscopically detectable tumor nodes were measured in animals once per week during weighing process. Two linear dimensions including the largest one and the largest rectangular to the former were recorded for each of the nodes. The largest dimension was taken as a length (a) and the second dimension as a width (b) of the tumor nodes. A tumor volume was calculated by the expression as follows:

$$V=(a\times b)2/2,$$

The efficacy of the therapy was assessed by recording a change in the average tumor volume, tumor growth inhibition (TGI), and the average total tumor volumes and its change over time in each of the mice.

The percent of tumor growth inhibition was calculated by the expression as follows:

$$TGI=(V_{ctrl}-V_{exp})/V_{ctrl}\times 100\ (\%),$$

where $V_{ctrl}$ is an average tumor volume in a control group, and $V_{exp}$ is an average tumor volume in an experimental group.

The primary data from individual forms were transferred to Microsoft Excel 2007 books. The group arithmetic average (M) and standard deviation (m) were calculated for all quantitative data. The statistical analysis was performed using the statistical software GraphPad Prism 6.0. The differences between the groups were assessed using ANOVA regression analysis and the Fisher's exact test.

When assessing the acute toxicity the animals were continuously observed for the first 60 minutes, and then examined every hour for 3 hours and further once in 24 hours. On the second day, the observation was carried out twice per day. Further the observations were made once per day. The clinical examination of each of the animals was carried

out after the active compound administration, every other day, and then weekly. The animal was thoroughly examined in the housing cage in the observer's hands. The overall conditions of the animals were recorded, such as peculiarities in their behavior, intensity and nature of motor activity, fur and skin appearance.

During the experiment no animals deaths were detected when the medicament was administered at a dose of 300 mg/kg. None of the animals exhibited visual signs of intoxication over the entire observation period. The appearance and behavior of the animals were as usual. When picked up by hands, the animals displayed a conventional weak reaction. No vocalization from the animals was detected upon the administration of the medicament or shortly thereafter.

The daily monitoring of the general condition and behavioral reactions of the animals has shown that a single oral administration of the test medicament did not affect the general condition and activity of the experimental animals exposed to intoxication.

Body weight data are shown in Tables 2 and 3.

Table 2.

| Changes in the body weights of the animals upon administration of the medicament at a dose of 300 mg/kg | | | | |
|---|---|---|---|---|
| 300 mg/kg dose | Days of experiment | | | |
| | 1 | 2 | 7 | 14 |
| 1 series | 03.05.2018 | 04.05.2018 | 09.05.2018 | 16.05.2018 |
| Mouse No. 1 | 26.4 | 26.8 | 27.2 | 27.6 |
| Mouse No. 2 | 29.6 | 29.2 | 29.6 | 30.2 |
| Mouse No. 3 | 27.8 | 27.4 | 28.2 | 29.0 |
| 2 series | 15.05.2018 | 16.05.2018 | 21.05.2018 | 28.05.2018 |
| Mouse No. 4 | 28.0 | 27.8 | 29.7 | 30.6 |
| Mouse No. 5 | 30.0 | 30.4 | 31.2 | 31.1 |
| Mouse No. 6 | 27.0 | 27.2 | 28.4 | 28.6 |
| Average (% from baseline) | 28.1 | 28.1 (0) | 29.1 (3) | 29.5(5) |
| Lethal effects (dead/total) | 0/6 | | | |

As follows from the data in Table 2, the average body weight of animals receiving the medicament at 300 mg/kg dose increased steadily during the observation period.

As no animals died upon administration of a dose of 300 mg/kg, the administration of the medicament was set at a dose of 2000 mg/kg. The medicament was administered in 2 steps with a break of 3 hours in a volume of 0.1 ml per 10 g of a body weight per step at the concentration of 100 mg/ml, because the suspension in oil is thicker at higher concentrations and precludes the medicament to be administered through a gavage. No clinical signs of intoxication were observed after the administration of 2000 mg/kg dose. Regardless of the group the width of the palpebral fissure of the animals was almost unchanged during the entire observation period. No pathological discharges from the eyes were detected. The nose was pink, moderately moist without pathological discharges. The fur of all mice was neat and shiny without bald spots. A decrease in the average body weight of animals was observed by 1% on Day 2 and by 2% on Day 7 from baseline (Table 3). The average body weight exceeded the baseline value by 3% at the end of the observation period.

Table 3.

| Changes in the body weight of the animals upon administration of the medicament at a dose of 2000 mg/kg dose | | | | |
|---|---|---|---|---|
| 2000 mg/kg dose | Days of experiment | | | |
| | 1 | 2 | 7 | 14 |
| 1 series | 30.05.2018 | 31.05.2018 | 05.06.2018 | 12.06.2018 |
| Mouse No. 1 | 27.2 | 26.4 | 26.3 | 27.0 |
| 1 series | 17.09.18 | 18.09.18 | 23.09.18 | 30.09.18 |
| Mouse No. 2 | 28.4 | 28.0 | 26.8 | 26.9 |

(continued)

| Changes in the body weight of the animals upon administration of the medicament at a dose of 2000 mg/kg dose | | | | |
|---|---|---|---|---|
| 2000 mg/kg dose | Days of experiment | | | |
| | 1 | 2 | 7 | 14 |
| Mouse No. 3 | 27.5 | 26.8 | 24.9 | 26.0 |
| 2 series | 30.09.18 | 01.10.18 | 06.10.2018 | 13.10.2018 |
| Mouse No. 4 | 25.7 | 25.3 | 25.0 | 26.6 |
| Mouse No. 5 | 24.2 | 24.4 | 25.9 | 28.7 |
| Mouse No. 6 | 24.0 | 23.8 | 25.4 | 26.9 |
| Average (% from baseline) | 26.2 | 25.8 (-1) | 25.7 (-2) | 27.0 (3) |
| Lethal effects (dead/ total) | 0/6 | | | |

All animals planned for euthanasia on Day 15 of the experiment were autopsied. Macroscopical examination did not reveal any abnormalities in the condition of internal organs.

The autopsy data were as follows:
The fur of the animals had a neat appearance, was shiny without bald spots. The nutritional status of the animals was satisfactory.

There were no pathological discharges from natural orifices.

The submandibular lymph nodes were rounded, pale pink in color, and moderately dense. The salivary glands were of normal shape, pale yellow in color, and moderately dense.

The peritoneum was smooth and shiny without free fluid in the cavity.

The spleen was not enlarged, dense, and the capsule was smooth and shiny. The pancreas was pale pink in color with lobed structure.

The size and shape of the liver were not changed, the liver capsule is shiny. The liver tissue had brownish color and moderately dense texture.

The kidneys were dense, the capsule was smooth, shiny, and there was a moderate outgrowth of adipose tissue around the kidneys.

The ovaries were not enlarged with shiny surface.

The stomach had folded shiny mucous membrane with a small amount of mucus and food contents in the lumen.

The pleura was smooth and shiny without free fluid in the chest cavity. The thymus was triangular in shape, whitish in color. Lungs were light pink in color, airy.

Therefore, according to the OECD 423 test, the medicament can be assigned to the $5^{th}$ or unclassified category with $LD_{50}$ being equal to or more than 5000 mg/kg, which corresponds to the class of low-hazard compounds.

The animals satisfactorily tolerated the administration of the medicament, no clinical signs of toxicity being observed. Moreover, the fur of animals receiving the medicament was shiny unlike the control group animals which had somewhat disheveled fur. In the control group, tumor nodes were also more evident.

No specific signs of toxic damage to internal organs were found after the autopsy analysis at the end of the experiment. The volume of tumor lesion was significantly smaller when the medicament was administered at 20 mg/kg dose and especially at 100 mg/kg dose compared to the control group animals.

The animals in all groups have gained the body weight during the entire observation period. The body weight gain might be associated with an increase in tumor node volumes in mice (Table 4).

Table 4.

| Changes in the average body weight of mice (g) when assessing the antitumor activity of the medicament | | | | | | |
|---|---|---|---|---|---|---|
| Group | Parameter | 18.09.18 (1) | 24.09.18 (7) | 01.10.18 (14) | 08.10.18 (21) | 15.10.18 (28) |
| Control-3 | M | 31.4 | 31.8 | 33.7 | 35.8 | 36.7 |
| | m | 0.9 | 1.1 | 1.2 | 1.4 | 1.5 |

(continued)

| Changes in the average body weight of mice (g) when assessing the antitumor activity of the medicament | | | | | | |
|---|---|---|---|---|---|---|
| 20 mg/kg | M | 31.9 | 31.8 | 33.5 | 35.2 | 36.7 |
| | m | 1.2 | 1.2 | 1.4 | 1.5 | 1.5 |
| 100 mg/kg | M | 31.4 | 31.7 | 32.0 | 33.7 | 34.4 |
| | m | 1.4 | 1.5 | 1.5 | 1.5 | 1.5 |
| Tamoxifen-2, 4.0 mg/kg# | Date | 20.08.18 (0) | 27.07.18 (7) | 03.09.18 (14) | 10.09.18 (21) | 17.09.18 (28) |
| | M | 31.0 | 32.0 | 32.9 | 32.8 | 34.2 |
| | m | 1.1 | 1.3 | 1.2 | 1.3 | 1.4 |

The analysis of changes in the average tumor volumes has shown that the administration of the medicament at 20 mg/kg and 100 mg/kg doses produces a statistically significant antitumor effect, thus TGI was 27% for 20 mg/kg dose and 42% for 100 mg/kg dose by Day 28 of the experiment; this effect was dose-dependent. It should be noted that the administration of 100 mg/kg led to the increase in the average tumor volume by only 60% from the baseline and by 155% in the control group during 28 days of the observation (Fig. 5).

Table 5.

| Changes of the average volume of tumors ($cm^3$) at the beginning of the experiment when assessing the antitumor activity of the medicament at the doses of 20 mg/kg and 100 mg/kg | | | | | | | |
|---|---|---|---|---|---|---|---|
| Group | Parameter | 17.09.18 (0) | 24.09.18 (7) | 01.10.18 (14) | 08.10.18 (21) | 15.10.18 (28) | % changes from baseline |
| Control-3 (N=60) | M | 0.38 | 0.5 | 0.7 | 0.85 | 0.97 | 155 |
| | m | 0.05 | 0.06 | 0.08 | 0.1 | 0.11 | |
| 20 mg/kg (N=49) | M | 0.35 | 0.39 | 0.55 | 0.64 | 0.71* | 103 |
| | m | 0.06 | 0.07 | 0.1 | 0.12 | 0.14 | |
| | TGI, % | 8 | 22 | 21 | 25 | 27 | |
| 100 mg/kg (N=48) | M | 0.35 | 0.37 | 0.48 | 0.57* | 0.56**a | 60 |
| | m | 0.04 | 0.03 | 0.04 | 0.05 | 0.05 | |
| | TGI, % | 8 | 26 | 31 | 33 | 42 | |
| Tamoxifen-2, 4 mg/kg# (N=39) | Date | 20.08.18 (0) | 27.07.18 (7) | 03.09.18 (14) | 10.09.18 (21) | 17.09.18 (28) | - |
| | M | 0.29 | 0.46 | 0.65 | 0.75 | 1.27* | 338 |
| | m | 0.04 | 0.07 | 0.08 | 0.09 | 0.16 | |
| | TGI, % | 24 | 8 | 7 | 12 | -31 | |
| M is the average value; m is a mean error; N is the number of tumors at the beginning of the treatment included in the analysis.<br>#the reference drug group is given from the third series of experiments.<br>*$p<0.05$ compared to the control group,<br>[a]$p<0.05$ compared to the reference drug group. | | | | | | | |

Notably, the administration of the medicament in each of the doses led to an increase in the tumor growth inhibition to the end of the observation period, but after the administration of the reference drug the TGI reached its maximum on Day 21 of the experiment. Therefore, the prolongation of the medicament administration is expected to increase the effect of the medicament.

The analysis of the stabilization frequencies has demonstrated a remarkable increase in this parameter from

8% in the control group up to 27% by the administration of the compound in 100 mg/kg dose. Moreover, the average volume of new tumors established during the observation period and their number were lower compared to the control group (Table 6).

Table 6.

| Frequency of tumor stabilizations, number and average volume of new tumors developed at the end of the experiment when assessing the antitumor activity of the medicament | | | | | |
|---|---|---|---|---|---|
| Group | Number of tumors at the beginning of the treatment and the frequency of their stabilizations | | Total number of new tumors | Average volume of new tumors at the end of observation period ($cm^3$) | |
| Control-3 | 60 | 8% | 25 | M | 0.23 |
| | | | | m | 0.04 |
| 20 mg/kg | 49 | 22% | 23 | M | 0.15 |
| | | | | m | 0.04 |
| 100 mg/kg | 48 | 27% | 15 | M | 0.12* |
| | | | | m | 0.02 |
| Tamoxifen-2, 4 mg/kg[#] | 39 | 3% | 32 | M | 0.20 |
| | | | | m | 0.05 |
| % of the disease stabilizations is a parameter similar to the RECIST criterion reflecting the frequency of tumors, which volume has not increased by no more than 20% by the end of observation in relation to the beginning of the treatment.<br>*p<0.05 compared to the control group.<br>[#]the reference drug group is given from the third series of experiments. | | | | | |

The average total volume of tumors in mice administered with the medicament at 20 mg/kg and 100 mg/kg doses was significantly lower as that of the control group from Days 14 to 28. For 100 mg/kg dose on Day 28 of the experiment, this parameter was statistically significantly lower as compared to the reference drug (Table 7, Fig. 6).

Table 7.

| The average total volume of tumors ($cm^3$) in mice when assessing the antitumor activity of the medicament. | | | | | | | |
|---|---|---|---|---|---|---|---|
| Group | Parameter | 17.09.18 (0) | 24.09.18 (7) | 01.10.18 (14) | 08.10.18 (21) | 15.10.18 (28) | % changes from baseline |
| Control-3 (N=10) | M | 2.26 | 3.02 | 4.17 | 5.07 | 5.80 | 157 |
| | m | 0.36 | 0.46 | 0.58 | 0.70 | 0.73 | |
| 20 mg/kg (N=10) | M | 1.69 | 1.93 | 2.67* | 3.15** | 3.48**[a] | 106 |
| | m | 0.30 | 0.33 | 0.47 | 0.56 | 0.65 | |
| | TGI, % | 25 | 36 | 36 | 38 | 40 | |
| 100 mg/kg (N=10) | M | 1.70 | 1.79 | 2.30* | 2.75** | 2.71**[a] | 59 |
| | m | 0.31 | 0.34 | 0.41 | 0.45 | 0.42 | |
| | TGI, % | 25 | 41 | 45 | 46 | 53 | |

(continued)

| The average total volume of tumors ($cm^3$) in mice when assessing the antitumor activity of the medicament. | | | | | | | |
|---|---|---|---|---|---|---|---|
| Tamoxifen-2, 4 mg/kg# (N=9) | Date | 20.08.18 (0) | 27.07.18 (7) | 03.09.18 (14) | 10.09.18 (21) | 17.09.18 (28) | - |
| | M | 1.26 | 2.01 | 2.84 | 3.23 | 5.52 | 338 |
| | m | 0.23 | 0.35 | 0.52 | 0.58 | 1.11 | |
| | TGI, % | 44 | 33 | 32 | 36 | 5 | |
| #the reference drug group is given from the third series of experiments.<br>*p<0.05 compared to the control group.<br>*p<0.05 compared to the reference drug group. | | | | | | | |

An assessment of the relative change in the average total tumor volume has demonstrated a dose-dependent effect of the medicament and efficacy superior of tamoxifen (Fig. 7, Table 8).

Table 8.

| The relative change of the average total volume to that of Day - 1 (expressed in %) in mice when assessing the antitumor activity of the medicament | | | | | | |
|---|---|---|---|---|---|---|
| Group | Parameter | 17.09.18 (0) | 24.09.18 (7) | 01.10.18 (14) | 08.10.18 (21) | 15.10.18 (28) |
| Control-3 | M | 0 | 34 | 88 | 131 | 175[a] |
| (N=10) | m | 0 | 6 | 14 | 22 | 25 |
| 20 mg/kg | M | 0 | 19 | 64[a] | 91[aa] | 107*[aa] |
| (N=10) | m | 0 | 6 | 16 | 17 | 20 |
| 100 mg/kg | M | 0 | 1[a] | 38[a] | 75[aa] | 69**aa |
| (N=10) | m | 0 | 7 | 10 | 16 | 16 |
| Tamoxifen-2, 4.0 mg/kg# | Date | 20.08.18 (0) | 27.07.18 (7) | 03.09.18 (14) | 10.09.18 (21) | 17.09.18 (28) |
| (N=9) | M | 0 | 67 | 134 | 178 | 363* |
| | m | 0 | 15 | 21 | 41 | 66 |

To estimate the hormonal effect of the medicament, the weight of uterine body without horns was measured on Day 28 of the observation period (Table 9). No significant differences of this parameter has been observed.

Table 9.

| Uterine body weight and body weight of mice with breast tumors when assessing the antitumor activity of the medicament | | | |
|---|---|---|---|
| Group | Uterine body weight (mg) | Estrous cycle phase (number of mice) | Body weight (g) |
| Control-3 | 27 | E - 1 | 28.6 |
| | 16±1 | D - 8 | 37.2±1.5 |
| | 17±2 | All | 36.7±1.5 |
| Tamoxifen-2, 4 mg/kg | 28±1 | E - 9 | 34.2± 1.4 |
| | - | D - 0 | - |
| | 28±1 | All | 34.2±1.4 |

(continued)

| Uterine body weight and body weight of mice with breast tumors when assessing the antitumor activity of the medicament | | | |
|---|---|---|---|
| Group | Uterine body weight (mg) | Estrous cycle phase (number of mice) | Body weight (g) |
| 20 mg/kg | 45±7 | E - 3 | 35.5±1.6 |
| | 19±3 | D - 7 | 37.1±2.2 |
| | 27±5 | All | 36.7±1.5 |
| 100 mg/kg | 50±10 | E - 2 | 31.5±5.4 |
| | 19±4 | D - 8 | 35.1±1.7 |
| | 26±6 | All | 34.4±1.5 |
| *including 1 animal in the metaestrus phase; E - estrus; D - diestrus. | | | |

The medicament at 20 mg/kg and 100 mg/kg doses has a pronounced antitumor effect, thus the TGI was 27% for 20 mg/kg dose and 42% for 100 mg/kg dose by Day 28 of the experiment, this effect was dose-dependent (differences were statistically significant). The administration at a dose of 100 mg/kg significantly increases the frequency of tumor stabilizations from 8% in the control group to 27%. The medicament at the dose of 100 mg/kg is statistically significantly more effective than the reference drug tamoxifen (4.0 mg/kg) on Day 28 to the end of the observation period.

Studies have shown that the medicament has no uterotropic activity.

According to the OECD 423 test the medicament can be assigned to the 5th or non-classified category with $LD_{50}$ being equal to or more than 5000 mg/kg, which corresponds to the class of low-hazard compounds according to the approved National State Standard.

## BRIEF DESCRIPTION OF DRAWINGS

The invention is illustrated by the following figures:

Fig. 1 depicts the staining of breast tumor with ERα antibodies.
Visualization of horseradish peroxidase + diaminobenzidine, magnification ×100 (A). A specific staining of breast duct cell nuclei and nuclei of single tumor cells (B), magnification ×400.
Fig. 2 depicts the average volume of tumors at the beginning of the experiment in mice receiving the CAF therapy.
Fig. 3 depicts the average total volume of tumors at the beginning of the experiment in mice receiving the CAF therapy.
Fig. 4 depicts the relative change in the average total volume of tumors established at the beginning of the experiment in mice receiving the CAF therapy.
Fig. 5 depicts the average volume of tumors in mice when assessing the antitumor activity of the medicament.
Fig. 6 depicts the average total volume of tumors in mice when assessing the antitumor activity of the medicament.
Fig. 7 depicts the relative change in the average total volume of tumors in mice when assessing the antitumor activity of the medicament.

## EXAMPLES

The invention can be further illustrated by examples of carrying out thereof disclosed herein below.

### Example 1.

The study analysis was carried out on an passaged MCF-7 human cells culture culture (breast adenocarcinoma). Normal human dermal fibroblasts (HDF) of early passages were used as a negative control. The cells were cultivated in Carrel vials in DMEM/F12 medium (Biolot) with 1.0 % of antibiotic-free fetal bovine embryonic serum (Biolot) added in the 5 % $CO_2$ atmosphere, at 37 °C.

The cells were seeded on Carrel vials at $50x10^4$ cells per flask. To study the proliferative activity of the cells under conditions of sulfatase inhibition, 24 hours after seeding the culture medium was replaced with the medium containing sulfatase inhibitors to a final concentration of 50 μg/ml, and then these tumor cell lines were incubated for various periods of time (24 to 72 hrs). The inhibitor was dissolved in DMSO. The final concentration of DMSO in the

culture medium did not exceed 0.5%. To exclude the cytotoxic effect of DMSO, a control sample comprising DMSO without sulfatase inhibitor was prepared. To exclude nonspecific detrimental effects of the compounds, normal human skin fibroblasts were used. Then the cells were detached with the versene-trypsin solution (Biolot), plated onto Carrel vials containing a fresh complete culture medium. The cells were counted when untreated control cells reached the maximum cell density per unit of surface area of a culture flask (monolayer), and their number was set as 100%. The proliferative activity of all of the studied cell cultures exposed to sulfatase inhibitors was determined in triplicate.

The study of the obtained sulfamate effects on the proliferation of passaged human MCF-7 cell culture (breast adenocarcinoma) has demonstrated that the steroid of formula (3) at a concentration of 20 μg/ml completely blocks the proliferation of tumor cells but it does not influence the growth of human skin fibroblasts having no estrogen receptors. The proliferation of tumor cells is inhibited to the same extent as under the action of Tamoxifen which has been used in clinical practice for more than 30 years. This is very important because sulfamates and tamoxifen have different mechanisms of action, and there is a potential for their use as a combination.

In the experiments on FVB mice, transgenic for HER-2/neu (ER-/PR-/HER2 +) having breast tumours, the agent inhibits the proliferation of tumours more effectively than clinically used Tamoxifen, which suggests the potential of their use as a combination for the treatment of breast cancer.

In triple negative breast cancer MDA-MB-231 cell line, the compound inhibits cell proliferation by 83%, which is similar to etoposide, a chemotherapeutic medicament used in clinical practice.

The medicament was active against hepatocellular carcinoma SK-Hep-1 cells ($IC_{50}$=53.4 μM), lung cancer A549 ($IC_{50}$=29.6 μM), gastric adenocarcinoma SNU638 ($IC_{50}$=22.8 μM), as well as against chronic myelogenous leukemia cells (K562).

**Example 2.**

*Control group.* There were 10 mice, 10 tumors per group. At the beginning of the therapy $V_{average}=1.5\pm0.4$ mm$^3$. Without a specific treatment tumor sizes reached $V_{average}=233.2\pm86.5$ mm$^3$ by Day 33 after transplantation. The number of dead mice in this group before the end of the observation period was 2 out of 10.

*Medicament group, 10 mg/kg.* There were 7 mice, 7 tumors per group.

At the beginning of the therapy $V_{average}=1.0\pm0.4$ mm$^3$ without significant differences from the control group. On Day 7 after the beginning of the treatment, non-significant TGI=74.3% was recorded ($V_{average}=5.1\pm2.3$ mm$^3$ versus $V_{average}=20.0\pm6.0$ mm$^3$ in the control, p<0.220). On Day 10 after the beginning of the treatment, TGI=68.0% ($V_{average}=24.8\pm12.2$ mm$^3$ versus $V_{average}=77.3\pm32.0$ mm$^3$ in the control, p<0.181). On Day 14 after the beginning of the treatment, the maximum significant TGI of 94.3% was observed ($V_{average}=6.9\pm2.2$ mm$^3$ versus $V_{average}=120.6\pm44.4$ mm$^3$ in the control, p<0.03) which remained at approximately the same level and was 90.6% ($V_{average}=17.6\pm0.7$ mm$^3$ versus $V_{average}=186.4\pm65.0$ mm$^3$ in control, p<0.061) and 90.2% ($V_{average}=26.1\pm12.1$ mm$^3$ versus $V_{average}=266.5\pm91.3$ mm$^3$ in control, p<0.061) by Day 17 and Day 21, respectively. The number of dead mice in this group before the end of the observation period was 5 out of 7.

*Tamoxifen group, 30 mg/kg.* There were 7 mice, 7 tumors per group. At the beginning of therapy $V_{average}=0.9\pm0.2$ mm$^3$ without significant differences from the control group. On Day 7, TGI was 12.6% ($V_{average}=18.8\pm7.4$ mm$^3$ versus $V_{average}=20.0\pm6$, mm$^3$ in the control, p<0.962) and did not exceed these values until the end of the observation period. On Day 17 and Day 21 after the beginning of the treatment, negative TGI was observed with a maximum value on Day 21 TGI=-44.2 ($V_{average}=384.8\pm103.2$ mm$^3$ versus $V_{average}=266.5\pm91.3$ mm$^3$ in control, p<0.106). The number of dead mice in this group before the end of the observation period was 6 out of 7.

*Tamoxifen group, 10 mg/kg.* There were 7 mice, 7 tumors per group. At the beginning of therapy $V_{average}=0.7\pm0.1$ mm$^3$ without significant differences from the control group. On Day 7 after the beginning of the treatment, non-significant TGI=63.9% was recorded ($V_{average}=7.2\pm3.9$ mm$^3$ versus $V_{average}=20.2\pm6.0$ mm$^3$ in the control, p<0.364). On Day 10 after the beginning of the treatment, TGI=56.9% was recorded ($V_{average}=33.4\pm16.1$ mm$^3$ versus $V_{average}=77.3\pm32.0$ mm$^3$ in the control, p<0.315). Through Day 14 to 17 an increase in TGI to 60.6% was observed ($V_{average}=47.5\pm16.8$ mm$^3$ versus $V_{average}=120.6\pm44.4$ mm$^3$ in the control, p<0.193) and 68.9% ($V_{average}=58.0\pm18.2$ mm$^3$ versus $V_{average}=186.4\pm65.0$ mm$^3$ in the control, p<0.070), respectively. On Day 21 after the beginning of the treatment and until the end of the observation period, TGI was below biologically significant values (<50%). The number of dead mice in this group before the end of the observation period was 2 out of 7.

The final results of the measurements and statistical significance of their differences between the groups are shown in Tables 10 and 11.

Table 10.

| The average tumor volumes with SEM in mice in the groups (M±m) | | | | | | | | |
|---|---|---|---|---|---|---|---|---|
| Groups | Days from the beginning of the treatment | | | | | | | |
| | 0 | 3 | 7 | 10 | 14 | 17 | 21 | 24 |
| Steroid, 10 mg/kg | 1.0±0. 4 | 1.2±0. 4 | 5.1+2.3 | 24.8±12. 2 | 6.9±2.2 | 17.6±0.7 | 26.2±12.1 | 32.0±6.9 |
| Tamoxifen 30 mg/kg | 0.9±0. 2 | 2.0±0. 4 | 18.8±7. 4 | 67.6±21. 1 | 117.1±41. 7 | 218.3±63. 6 | 384.8±103. 2 | 392.1 |
| Tamoxifen 10 mg/kg | 0.7±0. 1 | 1.0+0. 3 | 7.2±3.9 | 33.4±16. 1 | 47.5±16.8 | 58.0±18.2 | 145.5±70.9 | 211.1+94. 0 |
| Control | 1.5+0. 4 | 2.2+0. 5 | 20.0±6. 0 | 77.3±32. 0 | 120.6+44. 4 | 186.4±65. 0 | 266.5±91.3 | 233.2±86. 5 |

Table 11.

| Effects of the medicament and Tamoxifen on SKBR-3 growth inhibition over the entire observation period, TGI% | | | | | | | |
|---|---|---|---|---|---|---|---|
| Group | | Days from the beginning of the treatment | | | | | |
| | | 7 | 10 | 14 | 17 | 21 | 24 |
| 1 | Medicament. 10 mg/kg | 74.3 | 68.0 | 94.3 | 90.6 | 90.2 | 86.3 |
| 2 | Tamoxifen, 30 mg/kg | 6.2 | 12.6 | 2.9 | -17.1 | -44.4 | -68.1 |
| 3 | Tamoxifen, 10 mg/kg | 63.9 | 56.9 | 60.6 | 68.9 | 45.4 | 9.5 |

**Example 3.**

*Control group.* There were 12 mice, 12 tumors per group. At the beginning of the therapy $V_{average}$=3.8±1.0 $mm^3$. Without a specific treatment tumor sizes reached $V_{average}$=299.1±100.5 $mm^3$ by Day 30 after transplantation. The number of dead mice in this group before the end of the observation period was 6 out of 12.

*Medicament group, 10 mg/kg.* There were 10 mice, 10 tumors per group. At the beginning of therapy $V_{average}$=1.6±0.4 $mm^3$ without significant differences from the control group. On Day 7 after the beginning of the treatment, significant TGI=66.1% was recorded ($V_{average}$=27.0+5.1 $mm^3$ versus $V_{average}$=79.6±14.2 $mm^3$ in the control, p<0.002). On Day 10 after the beginning of the treatment, TGI=78.7% was observed ($V_{average}$=21.6±4.9 $mm^3$ versus $V_{average}$=101.2±22.5 $mm^3$ in the control, p<0.007). On Day 14 after the beginning of the treatment, the maximum non-significant TGI equal to 83.1% was observed ($V_{average}$=27.6±2.6 $mm^3$ versus $V_{average}$=163.3±31.0 $mm^3$ in control, p<0.06). On Day 17 after the beginning of the treatment, TGI decreased to 76.1% ($V_{average}$=46.3±5.3 $mm^3$ versus $V_{average}$=193.6±39.6 $mm^3$ in the control, p<0.04). The number of dead mice in this group before the end of the observation period was 10 out of 10.

*Tamoxifen group, 30 mg/kg.* There were 10 mice, 10 tumors per group. At the beginning of therapy $V_{average}$=4.1±1.2 $mm^3$ without significant differences from the control group. On Day 7 and until the end of the observation period, TGI was below a biologically significant threshold and did not exceed 44.5% ($V_{average}$=44.1±8.8 $mm^3$ versus $V_{average}$=79.6±14.2 $mm^3$ in the control, p<0.10). The number of dead mice in this group before the end of the observation period was 4 out of 10.

*Tamoxifen group, 10 mg/kg.* There were 10 mice, 10 tumors per group. At the beginning of therapy $V_{average}$=2.3±0.8 $mm^3$ without significant differences from the control group. In this group, no tumor growth inhibition was detected during the entire observation period. The number of dead mice in this group before the end of the observation period was 6 out of 9.

The results of the measurements and statistical significance of their differences between the groups are shown in Tables 12 and 13.

Table 12.

| The average tumor volumes with SEM in mice in the groups (M±m) | | | | | | | |
|---|---|---|---|---|---|---|---|
| Groups | Days from the beginning of the treatment | | | | | | |
| | 0 | 3 | 7 | 10 | 14 | 17 | 21 |
| Medicament , i.g. 10 | 1.57±0.35 | 5.65±1.64 | 26.98±5.12 | 21.59±4.88 | 27.59+2.57 | 46.25+5.31 | |
| Tamoxifen, 30 mg/kg (2) | 4.14±1.22 | 17.12±4.12 | 44.13±8.81 | 61.37±12.03 | 119.53±18.54 | 1141.85±35.07 | 199.54±43.55 |
| Tamoxifen, 10 mg/kg (3) | 2.33±0.76 | 25.61±5.56 | 79.17±18.80 | 122.09+27.84 | 193.24±54.10 | 237.32:1:77.07 | 322.76±88.25 |
| Control (4) | 3.84±1.04 | 28.52±6.27 | 79.57±14.20 | 101.19±22.51 | 163.25±31.00 | 193.58±39.56 | 299.05±100.50 |

Table 13.

| Effect of the medicament and Tamoxifen on TNBC_Kad growth inhibition over the entire observation period, TGI% | | | | | | |
|---|---|---|---|---|---|---|
| Group | | Days from the beginning of the treatment | | | | |
| | | 7 | 10 | 14 | 17 | 21 |
| 1 | Medicament, 10 mg/kg | 66.1 | 78.7 | 83.1 | 76.1 | - |
| 2 | Tamoxifen, 30 mg/kg | 44.5 | 39.4 | 26.8 | 26.7 | 33.3 |
| 3 | Tamoxifen, 10 mg/kg | 0.5 | -20.7 | -18.4 | -22.6 | -7.9 |

The carried out comparative studies of the antitumor activity of the medicament after multiple administrations to athymic immunodeficient Balb/c nude mice in two models of subcutaneous xenografts of human SKBR-3 and TNBC-Kad breast cancers which are different in phenotype.

A pilot experiment of the antitumor activity of the medicament in SKBR3 model in comparison with Tamoxifen (dosage form) has shown that the therapy with the medicament at a dose of 10 mg/kg was the most effective among all studied treatment regimens: on Day 10 after the beginning of the therapy, a significant antitumor effect was revealed, i.e. the maximum TGI=91.2% ($V_{average}$=2.2±2.0 $mm^3$ versus $V_{average}$=24.9±12.5 $mm^3$ in control, p<0.018) alongside with a relatively good tolerability of the medicament. Tamoxifen at 10 mg/kg dose was not effective: a biologically significant TGI value was not reached over the entire observation period. On the contrary, on Days 10 to 17 after the beginning of the treatment, the tumor growth stimulation was recorded.

A repeated experiment of the antitumor activity of the medicament in the same SKBR3 model in comparison with Tamoxifen (the reference drug) has shown that the medicament at 10 mg/kg dose has a high antitumor effect with maximum significant TGI on Day 14 (94.3%, p<0.03) and Day 24 (86.3%, p<0.04) that reproduced the result obtained in the first experiment.

An experiment of the antitumor activity of the medicament in TNBC_Kad model in comparison with Tamoxifen (the reference drug) has shown that the medicament at 10 mg/kg dose inhibits the tumor growth by 66.1 and 78.7% on Days 7 and 10 after the beginning of the treatment (the significant differences from the control groups and groups with tamoxifen 30 mg/kg being observed). The effect lasts for 10 days at approximately the same level. Tamoxifen at 30 mg/kg dose causes a weak non-significant inhibition of tumor growth by 44.5% on Day 7 which decreases to the end of observation. Tamoxifen at 10 mg/kg dose is not effective in this model.

**INDUSTRIAL APPLICABILITY**

The conducted experiments have shown that the claimed use of the compound is characterized by:

- the absence of uterotropic activity,
- a wide range of anti-cancer activities including activity against triple negative breast cancer,
- the absence of toxicity,
- the hypocholesterolemic activity and the absence of effects on the content of triglycerides in blood serum,
- the absence of negative effects on the endometrium.

The invention can be used for:

- the monotherapy and adjuvant therapy of early hormone-positive breast cancer in postmenopausal women;
- the monotherapy and adjuvant therapy of early stage of hormone-positive breast cancer in postmenopausal women having treated with tamoxifen for 2-3 years;
- the treatment of advanced breast cancer at late stages;
- the treatment of triple negative breast cancer;
- the monotherapy and adjuvant therapy of hepatocellular carcinoma;
- the monotherapy and adjuvant therapy of gastric carcinoma;
- the monotherapy and adjuvant therapy of lung cancer;
- the monotherapy and adjuvant therapy of chronic myelogenous leukemia.

## Claims

1. Use of 3-O-sulfamate-16,16-dimethyl-D-homoequilenin of formula (3)

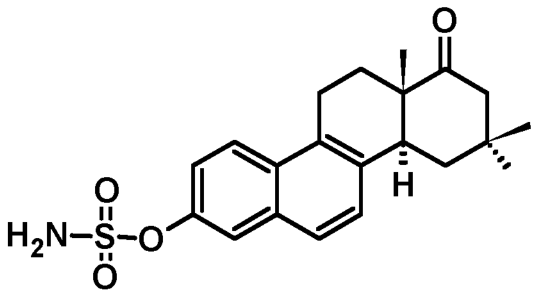

3

as an anti-cancer agent in monotherapy and adjuvant therapy of an oncological disease such as hepatocellular carcinoma;
gastric carcinoma;
lung cancer;
chronic myelogenous leukemia;
breast cancer;
triple negative form of breast cancer.

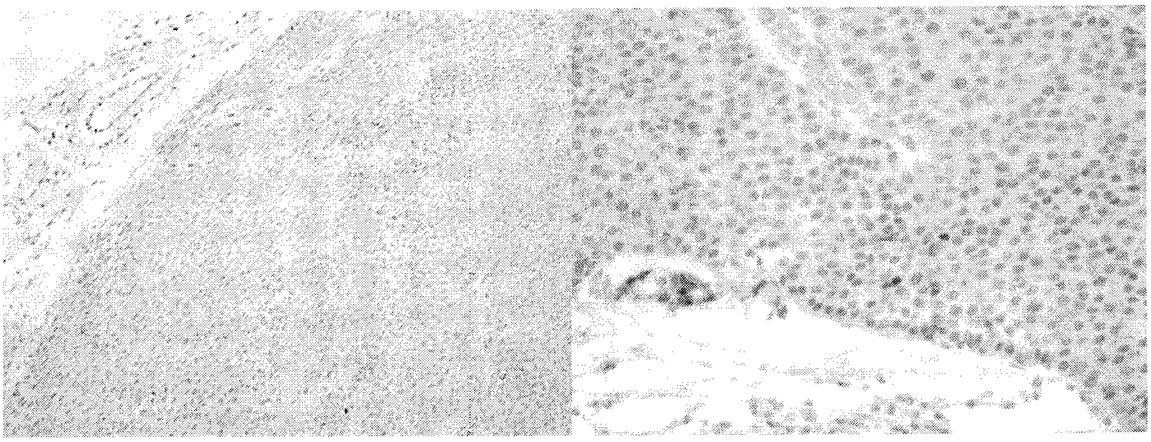

Fig. 1

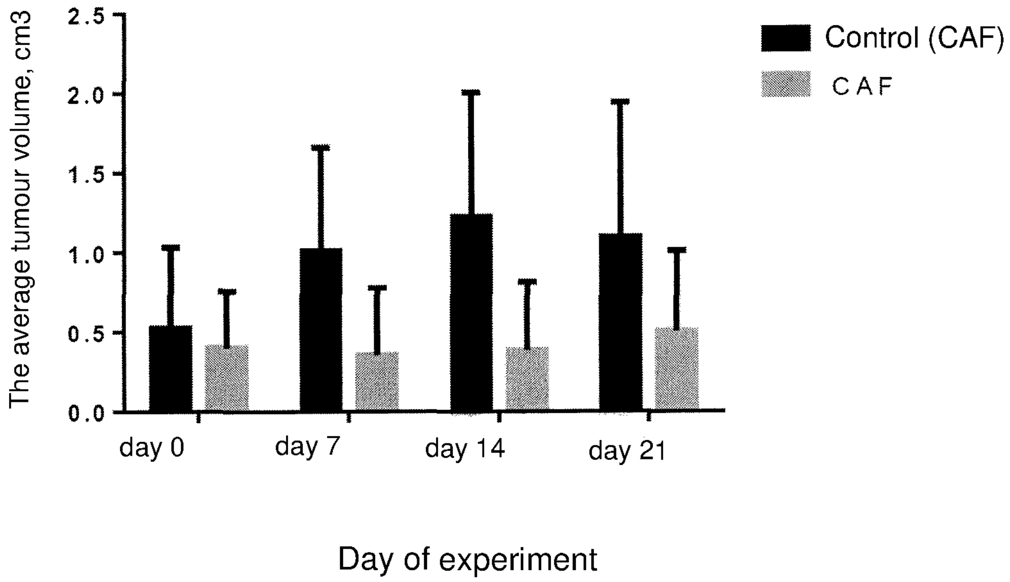
The average tumour volume, cm3
2.5
2.0
1.5
1.0
0.5
0.0
day 0
day 7
day 14
day 21
Day of experiment
Control (CAF)
CAF

Fig. 2

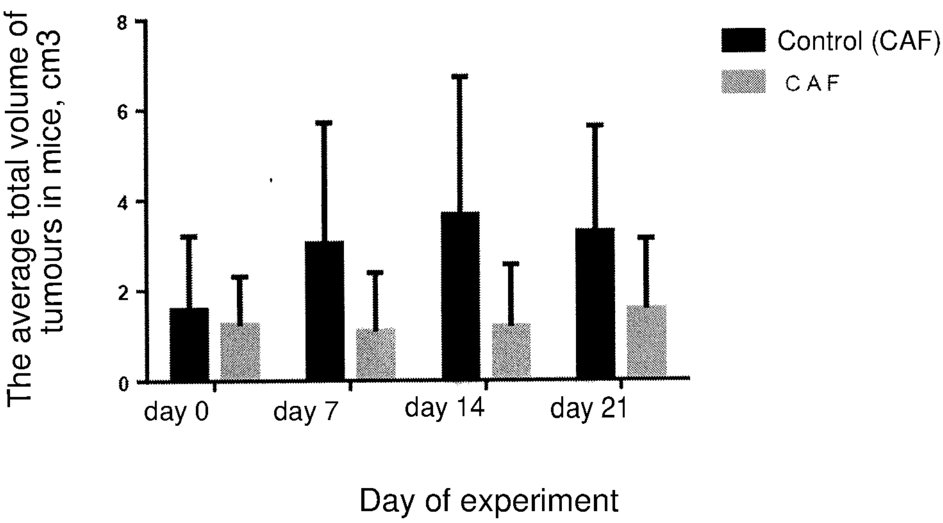
The average total volume of tumours in mice, cm3
8
6
4
2
0
day 0
day 7
day 14
day 21
Control (CAF)
CAF
Day of experiment

Fig. 3

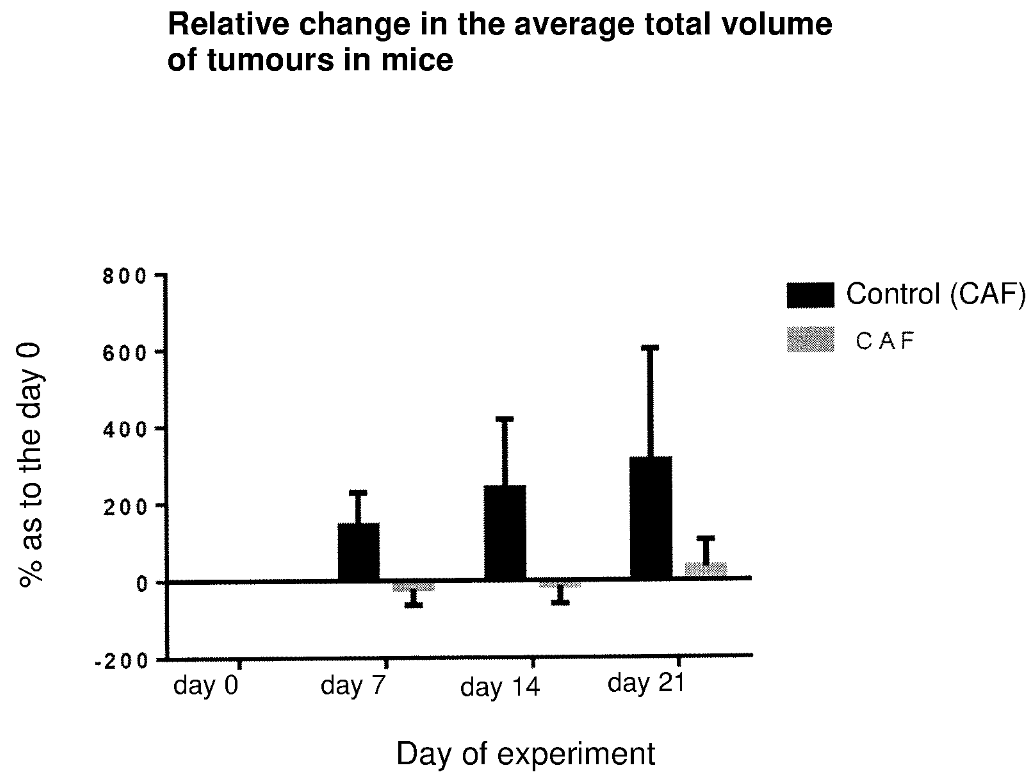
Relative change in the average total volume of tumours in mice
800
600
400
200
0
-200
% as to the day 0
day 0
day 7
day 14
day 21
Day of experiment
Control (CAF)
CAF

Fig. 4

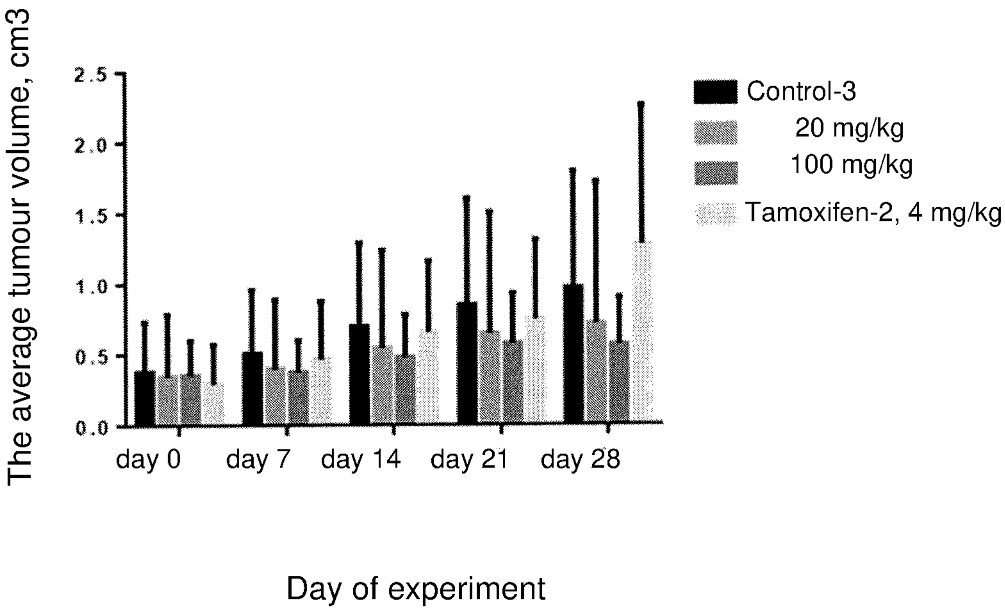
The average tumour volume, cm3
2.5
2.0
1.5
1.0
0.5
0.0
day 0
day 7
day 14
day 21
day 28
Control-3
20 mg/kg
100 mg/kg
Tamoxifen-2, 4 mg/kg
Day of experiment

Fig. 5

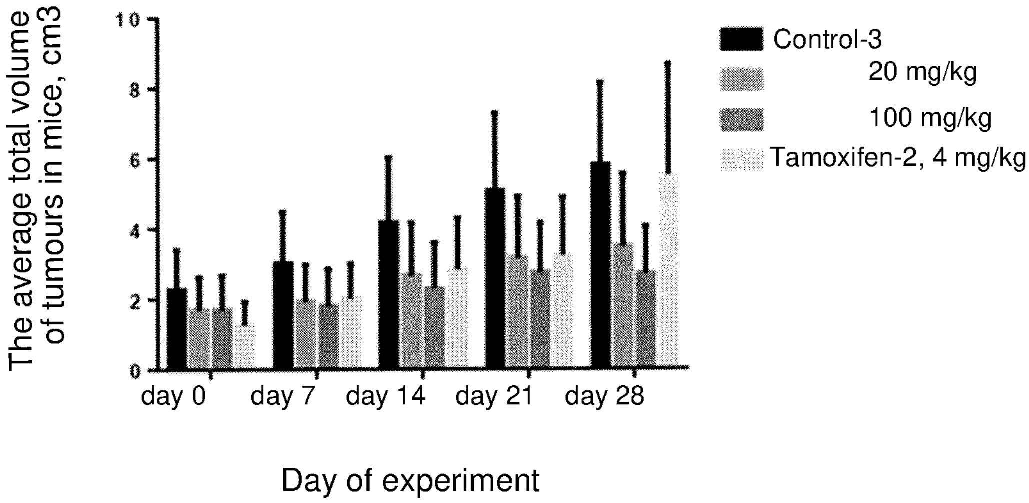
The average total volume of tumours in mice, cm3
10
8
6
4
2
0
day 0
day 7
day 14
day 21
day 28
Day of experiment
Control-3
20 mg/kg
100 mg/kg
Tamoxifen-2, 4 mg/kg

Fig. 6

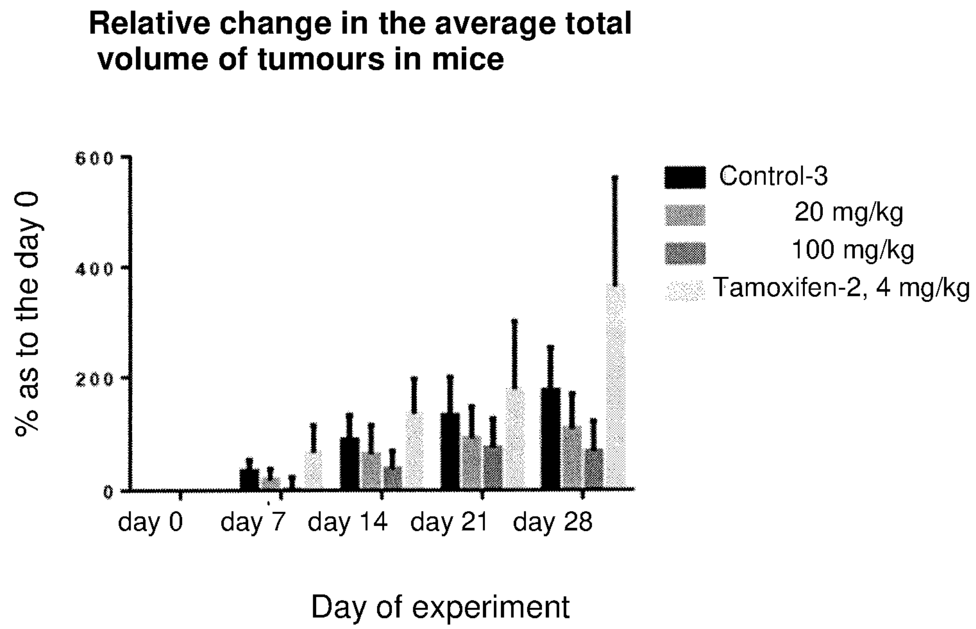
Relative change in the average total
volume of tumours in mice
% as to the day 0
600
400
200
0
day 0
day 7
day 14
day 21
day 28
Day of experiment
Control-3
20 mg/kg
100 mg/kg
Tamoxifen-2, 4 mg/kg

Fig. 7

## INTERNATIONAL SEARCH REPORT

International application No.
PCT/RU 2019/000650

A. CLASSIFICATION OF SUBJECT MATTER

A61K 31/566 (2006.01); A61P 35/00 (2006.01); C07J 63/00 (2006.01)

According to International Patent Classification (IPC) or to both national classification and IPC

B. FIELDS SEARCHED

Minimum documentation searched (classification system followed by classification symbols)

A61K 31/566, A61P 35/00, C07J 63/00

Documentation searched other than minimum documentation to the extent that such documents are included in the fields searched

Electronic data base consulted during the international search (name of data base and, where practicable, search terms used)

PatSearch (RUPTO internal), USPTO, PAJ, Esp@cenet, DWPI, EAPATIS, PATENTSCOPE

C. DOCUMENTS CONSIDERED TO BE RELEVANT

| Category* | Citation of document, with indication, where appropriate, of the relevant passages | Relevant to claim No. |
|---|---|---|
| X | GLUZDIKOV I. A. Sintez ingibitorov sulfatazy estrona: Avtoref. disp. na soiskanie uchenoi stepeni kandidata khimicheskikh nauk. Sankt-Peterburg: 2007, 17 p., compound 6 | 1 |
| A | I. A. Gluzdikov et al. "Novye analogi D-gomoekvilenina, soderzhaschie zamestiteli v koltse D" ZHURNAL ORGANICHESKOI KHIMII, V. 42, Issue 11, 2006, 41687-1694 | 1 |
| A | S.N. Morozkina et al. "Sintez i issledovanie nekotorykh biologicheskikh svoistv sulfamatov 8 a- analogov steroidnykh estrogenov" ZHURNAL ORGANICHESKOI KHIMII, V. 51, Issue 3, 2015, 425-430 | 1 |
| A | WO 2004/074309 A1 (SCHERING AKTIENGESELLSCHAFT) 02.09.2004 | 1 |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 20 December 2019 (20.12.2019) | 09 January 2020 (09.01.2020) |
| Name and mailing address of the ISA/ RU | Authorized officer |
| Facsimile No. | Telephone No. |

Form PCT/ISA/210 (second sheet) (July 1998)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PARKIN D.M. ; BRAY F. ; FERLAY J. ; PISANI P.** *CA Cancer J. Clin.,* 2005, vol. 55, 74-108
- **YUE W. ; YAGER J.D. ; WANG J.-P. ; JUPE E.R. ; SANTEN R.J.** *Steroids,* 2013, vol. 78, 161-170
- **DIBBELT I.** *Biol. Chem., Hoppe-Seyler,* 1991, vol. 372, 173-185
- **STEIN C.** *J. Biol. Chem.,* 1989, vol. 264, 13865-13872
- **AHMED S.** *Curr. Med. Chem.,* 2002, vol. 9 (2), 263-273
- **HOWARTH N.M. ; PUROHIT A. ; REED M.J.** *J. Med. Chem.,* 1994, vol. 37, 219-221
- **SHIELDS-BOTELLA J. et al.** *J. SteroidBiochem. Mol. Biol.,* 2003, vol. 84, 327-335
- **URUSOVA E.A. ; GLUZDIKOV I.A. ; SELIVANOV S.I. ; STAROVA G.L. ; NIKOLAEV S.V. ; SHAVVA A.G.** Synthesis and study of equilenin derivatives and its modified analogs. *Russ. J. Org. Chem.,* 2004, vol. 40 (4), 506-512
- **AL SAFAR ; ZAKHARYCHEV A.V. ; ANANCHENKO S.N. ; TORGOV I.V.** Synthesis of some 16,16-dimethyl-D-homosteroids. *Reports of Academy of Sciences of the USSR. Ser. Chem.,* 1968, vol. 10, 2326-2332
- **GLUZDIKOV I.A. ; EGOROV M.S. ; SELIVANOV S.I. ; STAROVA G.L. ; SHAVVA A.G.** New analogues of D-homoequilenene with substituents in the D ring. *Rus. J. Org. Chem.,* 2006, vol. 42 (11), 1675-1682
- **GLUZDIKOV I.A.** *PhD Thesis in Chemistry,* 2007
- **MOROZKINA S.N. ; GLUZDIKOV I.A. ; DROZDOV A.S. ; SELIVANOV S.I. ; KOVALEV R.A. ; FILATOV M.V. ; SHAVVA A.G.** *Russ. J. Org. Chem.,* 2015, vol. 51 (3), 425-430
- The Guide for Care and Use of Laboratory Animals. ILAR. National Academy Press, 1996